# EUROPEAN PATENT APPLICATION

(11) **EP 4 137 141 A1**
(43) Date of publication of application: **22.02.2023**
(21) Application number: 21788756.1
(22) Date of filing: 09.04.2021
(51) Int. Cl.: A61K 35/28, A61K 47/20, A61P 13/12, A61P 37/02

(54) **AGENT FOR INCREASING CD25-POSITIVE REGULATORY T CELLS IN KIDNEY**

(30) Priority: 13.04.2020 JP 2020071737
(71) Applicant: National University Corporation Tokai National Higher Education and Research System, Nagoya-shi, Aichi 464-8601 (JP); Rohto Pharmaceutical Co., Ltd., Osaka-shi, Osaka 544-8666 (JP)
(72) Inventor: MARUYAMA Shoichi, Nagoya-shi, Aichi 464-8601 (JP); FURUHASHI Kazuhiro, Nagoya-shi, Aichi 464-8601 (JP); TANAKA Akihito, Nagoya-shi, Aichi 464-8601 (JP); KARASAWA Munetoshi, Nagoya-shi, Aichi 464-8601 (JP); TAKAO Yukinari, Osaka-shi, Osaka 544-8666 (JP)
(74) Representative: Mewburn Ellis LLP
(86) International application number: PCT/JP2021/015093
(87) International publication number: WO 2021/210515

(57) **Abstract**

The present invention addresses the problem of providing a fundamental therapeutic method for renal diseases including glomerulonephritis such as IgA nephropathy. To solve this problem, the present invention pertains to an agent for increasing CD25-positive regulatory T cells in kidney, said agent comprising mesenchymal stem cells. The regulatory T cell-increasing agent of the present invention exerts a sufficient therapeutic effect even if further containing a cryopreservation liquid at a concentration of less than 5% (v/v).

## Description

### TECHNICAL FIELD

The present invention relates to an agent for increasing CD25-positive regulatory T cells in kidneys.

### BACKGROUND ART

IgA nephropathy is a chronic primary glomerulonephritis in which a deposit mainly containing IgAis observed in mesangial regions of glomeruli. It is surmised that some antigen promotes the production of an IgA antibody, an immune complex (IC) in which the antigen and the antibody are bound deposits on glomeruli, and glomerulonephritis is caused. Since, as to treatment for this disease, fundamental therapy has not been obtained, symptomatic therapies such as renin-angiotensin system inhibitors, adrenocortical steroid drugs (pulse therapies), immunosuppressive drugs, and tonsillectomy are performed, and the development of a therapy therefor has been desired.

Mesenchymal stem cells are cells having pluripotency and first isolated from bone marrow by Friedenstein (refer to Non Patent Document 1). Since mesenchymal stem cells have the ability to differentiate to bones, cartilage, and adipose, mesenchymal stem cells attract attention as a promising cell source in cell therapies. It is known recently that cells having equivalent function are present among mesenchymal stromal cells that are an adipose tissue and fetal appendages such as a placenta, an umbilical cord, and a vitelline coat. Therefore, mesenchymal stem cells may be referred to as mesenchymal stromal cells. Examples in which mesenchymal stem cells are used for treatment for diseases including treatment for acute graft-versus-host disease (GVHD) in hematopoietic stem cell transplantation (refer to Non Patent Document 2), treatment for Crohn disease, which is inflammatory bowel disease, and treatment for cerebral infarction (refer to Non Patent Document 3).

### PRIOR ART DOCUMENTS

### Non Patent Document

Non Patent Document 1: Pittenger F. M.et al. Science, (1999), 284, pp.143-147
Non Patent Document 2: Designated regenerative medical product Temcell (R) HS Inj. attached document, January, 2017
Non Patent Document 3: Regenerative medical product Stemirac (R) for Injection attached document, December, 2018

### SUMMARY OF INVENTION

### Technical Problem

An object of the present invention is to provide fundamental therapy for renal diseases including glomerulonephritis such as IgA nephropathy in the above-mentioned situation.

### Solution to Problem

The present inventors have earnestly examined to achieve the above-mentioned object and consequently found that it is effective for glomerulonephritis to increase CD25-positive regulatory T cells in kidneys. The present invention has been completed based on this knowledge.

That is, the invention completed to achieve the above-mentioned object relates to an agent for increasing CD25-positive regulatory T cells in kidneys.
[1] An agent for increasing CD25-positive regulatory T cells in kidneys, comprising mesenchymal stem cells.
[2] The agent for increasing CD25-positive regulatory T cells in kidneys according to [1], further comprising a cryopreservation solution at a concentration of less than 5% (v/v).
[3] The agent for increasing CD25-positive regulatory T cells in kidneys according to [2], wherein the cryopreservation solution is a cryopreservation solution comprising DMSO (dimethyl sulfoxide).
[4] The agent for increasing CD25-positive regulatory T cells in kidneys according to any one of [1] to [3], wherein the agent is for treating a renal disease.
[5] The agent for increasing CD25-positive regulatory T cells in kidneys according to [4], wherein the renal disease is glomerulonephritis.
[6] A method for increasing CD25-positive regulatory T cells in kidneys, comprising administering mesenchymal stem cells.
[7] A method for treating a renal disease, comprising administering an agent for increasing CD25-positive regulatory T cells to increase CD25-positive regulatory T cells in kidneys.
[8] The method for treating a renal disease according to [7], wherein the agent for increasing CD25-positive regulatory T cells comprises mesenchymal stem cells.
[9] The method for treating a renal disease according to [7] or [8], wherein the renal disease is glomerulonephritis.

### Advantageous Effects of Invention

Since CD25-positive regulatory T cells in kidneys can be increased according to an agent for increasing regulatory T cells of the present invention, the function, structure, and the like of disease sites of the kidney or the like can be improved remarkably. An excellent therapeutic effect is expected to be produced on renal diseases including glomerulonephritis such as IgA nephropathy thereby. Since mesenchymal stem cells contained in an agent for increasing regulatory T cells of the present invention are hardly rejected by an allogeneic subject, cells obtained by subjecting donor cells prepared in advance to expansion culture and cryopreservation can be used. Therefore, it is also advantageous that the mesenchymal stem cells of the present invention are also easily commercialized compared to when autologous mesenchymal stem cells are prepared and used, and a certain stable effect can be easily obtained.

### BRIEF DESCRIPTION OF DRAWINGS

[Fig. 1] Fig. 1 shows the measurement results of the serum creatinine concentration (left figure), the blood urea nitrogen (central figure), and the crescent score (right figure) of rats in which nephritis develops 3 days after the final administration of mesenchymal stem cells to anti-GBM nephritis rats.
[Fig. 2] Fig. 2 shows the measurement results of the serum creatinine concentration (left figure) and the blood urea nitrogen (right figure) of anti-GBM nephritis rats 3 days after the final administration of mesenchymal stem cells.
[Fig. 3] Fig. 3 shows the measurement result of the serum creatinine concentration (left figure) and the blood urea nitrogen (right figure) of anti-GBM nephritis rats 3 days after the final administration of mesenchymal stem cells.
[Fig. 4] Fig. 4 shows the measurement result of the serum creatinine concentration (left figure) and the blood urea nitrogen (right figure) of anti-GBM nephritis rats 3 days after the final administration of mesenchymal stem cells.
[Fig. 5] Fig. 5 shows the rate of CD25-positive Treg cells among CD45-positive cells in kidneys of anti-GBM nephritis rats 3 days after the final administration of mesenchymal stem cells.
[Fig. 6] Fig. 6 shows the results obtained by comparing the rate of CD25-positive Treg cells among CD45-positive cells in kidneys of anti-GBM nephritis rats and the serum creatinine concentration 3 days after the final administration of mesenchymal stem cells.

### DESCRIPTION OF EMBODIMENTS

Hereinafter, an agent for increasing regulatory T cells of the present invention will be described in detail.

### <Agent for increasing regulatory T cells>

The agent for increasing regulatory T cells of the present invention is characterized by containing mesenchymal stem cells.

### [Regulatory T cells (Treg cells)]

T cells are cells that has proteins called T cell receptors on the cell surface, recognizes foreign substances through these receptors specifically, and are activated. T cells are roughly classified by the function thereof into killer T cells that kill virus-infected cells, cancer cells, and the like specifically and helper T cells that work on other immunocytes such as B cells and macrophages (phagocytes), and activate the functions thereof. Regulatory T cells (Treg cell) are one type of T cells, and are cells having the function of working on immunocytes such as killer T cells, helper T cells, B cells, and macrophages and suppressing the activation thereof. Regulatory T cells have the function of suppressing an immune response, and suppresses excessive immune response that causes autoimmune diseases, inflammatory diseases, allergic diseases, or the like. In the present invention, it is determined that T cells in which the surface antigen expression is CD45⁺CD3⁺CD4⁺CD8⁻CD25⁺Foxp3⁺ are regulatory T cells (Treg cells).

### [Mesenchymal stem cells]

In the present invention, mesenchymal stem cells mean cells that have the ability to differentiate to cells belonging to mesenchymal cells (osteocytes, cardiomyocytes, chondrocytes, tendon cells, adipocytes, and the like), and are capable of proliferating while maintaining the ability. The term mesenchymal stem cells used in the present invention means the same cells as mesenchymal stromal cells, and both are not particularly distinguished. Examples of a tissue containing mesenchymal stem cells include adipose tissue, umbilical cords, bone marrow, umbilical blood, endometria, placentae, dermis, skeletal muscle, periostea, dental follicles, periodontal membrane, dental pulp, and tooth germs. For example, mesenchymal stem cells derived from an adipose tissue therefore mean mesenchymal stem cells contained in an adipose tissue, and may be called an adipose tissue-derived mesenchymal stromal cells. Of these, adipose tissue-derived mesenchymal stem cells, umbilical cord-derived mesenchymal stem cells, bone marrow-derived mesenchymal stem cells, placenta-derived mesenchymal stem cells, and dental pulp-derived mesenchymal stem cells are preferable, adipose tissue-derived mesenchymal stem cells, bone marrow-derived mesenchymal stem cells, and umbilical cord-derived mesenchymal stem cells are more preferable, adipose tissue-derived mesenchymal stem cells and bone marrow-derived mesenchymal stem cells are further preferable, and adipose tissue-derived mesenchymal stem cells are particularly preferable.

It is preferable that the mesenchymal stem cells in the present invention be autologous or allogeneic to a subject. Since the mesenchymal stem cells are hardly rejected by an allogeneic subject, cells obtained by subjecting donor cells prepared in advance to expansion culture and cryopreservation can be used as mesenchymal stem cells in the therapeutic agent of the present invention. It is therefore more preferable from the viewpoints that the mesenchymal stem cells of the present invention are also easily commercialized compared to when autologous mesenchymal stem cells are prepared and used, and a certain effect can be easily and stably obtained that the mesenchymal stem cells in the present invention be allogeneic.

The mesenchymal stem cells in the present invention may be characterized by, for example, growth characteristics (for example, population doubling ability and doubling time from subculture to aging), karyotype analysis (for example, normal karyotype, maternal lineage or newborn lineage), surface marker expression analyzed by flow cytometry (for example, FACS analysis), immunohistochemistry and/or immunocytochemistry (for example, epitope detection), gene expression profiling (for example, gene chip array; polymerase chain reaction such as reverse transcription PCR, real time PCR, conventional PCR), miRNA expression profiling, protein array, the secretion of a protein such as a cytokine (for example, plasma coagulation analysis, ELISA, cytokine array), metabolites (metabolome analysis), other methods known in this field, or the like.

The mesenchymal stem cells can be prepared in accordance with a method known to a person skilled in the art. As an example, adipose tissue-derived mesenchymal stem cells can be prepared in accordance with a production method described, for example, in US Patent No. 6,777,231.

As long as the mesenchymal stem cells in the present invention have the effect of increasing CD25-positive regulatory T cells and the effect of treating disease, the mesenchymal stem cells may be cells appropriately cryopreserved and thawed repeatedly. In the present invention, cryopreservation can be carried out by suspending the mesenchymal stem cells in a cryopreservation solution known to a person skilled in the art and cooling the cells. Suspension can be carried out by peeling cells with a peeling agent such as trypsin, pouring the cells into a cryopreservation container, appropriately treating the cells, and then adding a cryopreservation solution.

A cryopreservation solution containing DMSO (dimethyl sulfoxide) as a cryoprotective agent is exemplified as the cryopreservation solution. When DMSO is used, the concentration of DMSO is 5% to 20%, preferably 5% to 10%, and more preferably 10%. As such a cryopreservation solution, for example, cryopreservation solutions provided from BioVerde Corporation, NIPPON Genetics Co, Ltd., REPROCELL Inc., NIPPON ZENYAKU KOGYO CO., LTD., Cosmo Bio Co., Ltd., Kohjin Bio Co., Ltd., Thermo Fisher Scientific K.K., and the like may be used.

The cryopreserved mesenchymal stem cells are thawed just before use, and can be directly mixed in a solution such as an infusion or a medium with the thawed cells suspended in a cryopreservation solution to prepare an agent for increasing regulatory T cells for use. The cryopreservation solution is removed by centrifugation or the like, and the resultant cells can also be then suspended in a solution such as an infusion or a medium to prepare an agent for increasing regulatory T cells for use. The concentration of the cryopreservation solution in the agent for increasing regulatory T cells is less than 5% (v/v), preferably less than 2% (v/v), and further preferably 1.65% (v/v) or less. Here, the "infusion" in the present invention refers to a solution to be used at the time of treating humans. Examples of the infusion include, but are not particularly limited to, physiological saline, Japanese Pharmacopoeia isotonic sodium chloride solution, a 5% glucose solution, Japanese Pharmacopoeia glucose injection solution, Ringer's solution, Japanese Pharmacopoeia Ringer's solution, Ringer's lactate solution, Ringer's acetate solution, No. 1 solution (starting solution), No. 2 solution (dehydrated replenisher), No. 3 solution (maintaining solution), and No. 4 solution (postoperative recovery solution).

The cryopreserved cells can be thawed by a method known to a person skilled in the art. A method for thawing the cryopreserved cells, for example, by leaving the cells to stand or shaking the cells in a constant temperature bath or in a hot water bath at 37°C is exemplified.

The agent for increasing regulatory T cells of the present invention may contain a pharmaceutically acceptable carrier and additives in accordance with a usual method depending on the use or form thereof as long as the effect of the present invention is not deteriorated.

The agent for increasing regulatory T cells of the present invention can be suspended or diluted using an infusion such as physiological saline, Japanese Pharmacopoeia isotonic sodium chloride solution, a 5% glucose solution, Japanese Pharmacopoeia glucose injection solution, Ringer's solution, Japanese Pharmacopoeia Ringer's solution, Ringer's lactate solution, Ringer's acetate solution, Ringer's bicarbonate solution, No. 1 solution (starting solution), No. 2 solution (dehydrated replenisher), No. 3 solution (maintaining solution), or No. 4 solution (postoperative recovery solution) or a cell culture medium such as DMEM and used.

The agent for increasing regulatory T cells of the present invention can be preferably used for treatment for diseases of kidneys (for a renal disease treatment). Especially, it can be more preferably used for treating glomerulonephritis such as acute glomerulonephritis and chronic glomerulonephritis; interstitial nephritis; an acute nephritic syndrome; recurrent and persistent hematuria; a chronic nephritic syndrome; a nephrotic syndrome; IgA nephropathy; a lupus nephritis; or an acute progressive nephritic syndrome.

Although the content of mesenchymal stem cells in the agent for increasing regulatory T cells of the present invention can be adjusted depending on the state (body weight, age, symptom, physical condition, or the like) of the patient and the dosage form of the agent for increasing regulatory T cells of the present invention, it tends to be preferable that the amount thereof be larger from the viewpoint of producing enough effect of increasing regulatory T cells in kidneys and enough effect of treating diseases. Meanwhile, it tends to be preferable that the amount thereof be smaller from the viewpoint of suppressing side effects. The dose in the case of administration to an adult is usually 1 × 10³ to 1 × 10¹² cells/time, preferably 1 × 10⁴ to 1 × 10¹¹ cells/time, more preferably 1 × 10⁵ to 1 × 10¹⁰ cells/time, and further preferably 5 × 10⁶ to 1 × 10⁹ cells/time in terms of the number of cells. The dose per body weight of a patient is 1 × 10 to 5 × 10¹⁰ cells/kg, preferably 1 × 10² to 5 × 10⁹ cells/kg, more preferably 1 × 10³ to 5 × 10⁸ cells/kg, and further preferably 1 × 10⁴ to 5 × 10⁷ cells/kg. Note that when this dose is defined as the amount per administration, the cells may be administered a plurality of times, or this dose may be divided into a plurality of portions, and the divided portions may be administered.

The agent for increasing regulatory T cells of the present invention may be administered with one or more additional drugs. Examples of the additional drugs include any drug that can be used as a therapeutic agent for the kidney, and specifically include immunosuppressive drugs such as adrenocortical steroid, mizoribine, cyclosporin, tacrolimus, and cyclophosphamide; antiplatelet drugs such as dipyridamole and dilazep hydrochloride hydrate; anticoagulant drugs such as heparin and warfarin potassium; hypotensive drugs such as an angiotensin converting enzyme (ACE) inhibitor, an angiotensin II receptor blocker (ARB) and a calcium antagonist; loop diuretics such as furosemide and azosemide; a concentrated albumin preparation; and a drug for improving dyslipidemia.

### <Method for increasing CD25-positive regulatory T cells in kidneys>

The present inventors have found out that it is effective in glomerulonephritis to increase CD25-positive regulatory T cells in kidneys, and further found that mesenchymal stem cells can increase CD25-positive regulatory T cells in kidneys. The present invention also includes a method for increasing CD25-positive regulatory T cells in kidneys, characterized by comprising administering mesenchymal stem cells. This method can also be referred to as a method for increase CD25-positive regulatory T cells in kidneys, characterized by comprising administering the above-mentioned agent for increasing regulatory T cells in other words. As specific description of the mesenchymal stem cells, CD25-positive regulatory T cells, and the agent for increasing regulatory T cells, the description in the section of the agent for increasing regulatory T cells is applicable.

### <Method for treating renal diseases>

The present invention includes a method for treating renal diseases, characterized by comprising administering the agent for increasing CD25-positive regulatory T cells to increase CD25-positive regulatory T cells in kidneys. The agent for increasing CD25-positive regulatory T cells preferably contains mesenchymal stem cells. As specific description of the mesenchymal stem cells, CD25-positive regulatory T cells, and the agent for increasing regulatory T cells, the description in the section of the agent for increasing regulatory T cells is applicable. Examples of the renal disease include glomerulonephritis such as acute glomerulonephritis and chronic glomerulonephritis; interstitial nephritis; an acute nephritic syndrome; recurrent and persistent hematuria; a chronic nephritic syndrome; a nephrotic syndrome; IgA nephropathy; a lupus nephritis; or an acute progressive nephritic syndrome. The therapeutic method of the present invention is effective in glomerulonephritis such as acute glomerulonephritis and chronic glomerulonephritis among these diseases.

### EXAMPLES

Although the present invention will be described in the following Examples specifically, the present invention is not limited by the Examples.

### EXAMPLE 1

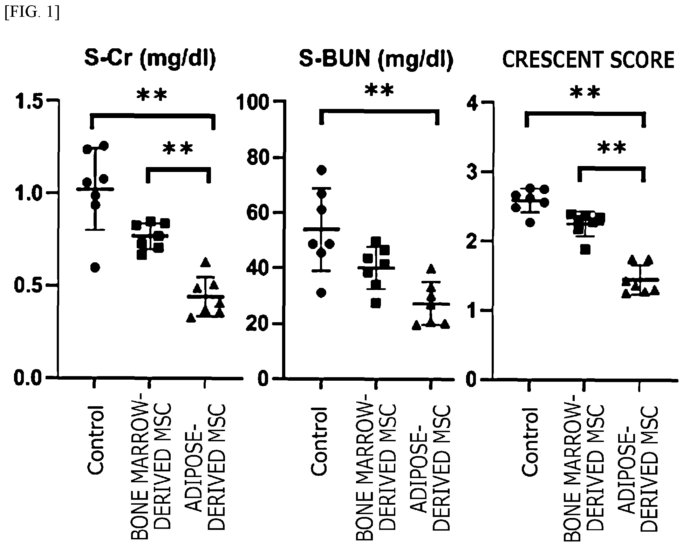

Anti-GBM mouse IgG (TF78), which was specifically bound to α4(IV)NC1 of rat glomerular basement membranes (GBMs), was administered to each rat (WKY/NCrj, female) to induce anti-glomerular basement membrane nephritis (Goodpasture syndrome, anti-GBM nephritis). Then, 2 × 10⁶ bone marrow-derived mesenchymal stem cells (produced by LONZA K. K., bone marrow-derived MSC group) or 2 × 10⁶ adipose-derived mesenchymal stem cells (produced by ROHTO Pharmaceutical Co., Ltd., adipose-derived MSC group) were intravenously administered to the rat on the second day and the fourth day immediately after the anti-GBM mouse IgG administration. The cells were suspended in 2 mL of HBSS and used for administration, and only HBSS was administered to a control group (control group). The serum creatinine concentration (S-Cr), the blood urea nitrogen (S-BUN) and the crescent score were measured on the seventh day. The administration of the bone marrow-derived MSCs and the adipose-derived MSCs suppressed creatinine concentration increase, blood urea nitrogen increase, and crescent score caused by anti-GBM nephritis; and those were remarkably suppressed by administering especially adipose-derived MSCs (Fig. 1).

### EXAMPLE 2

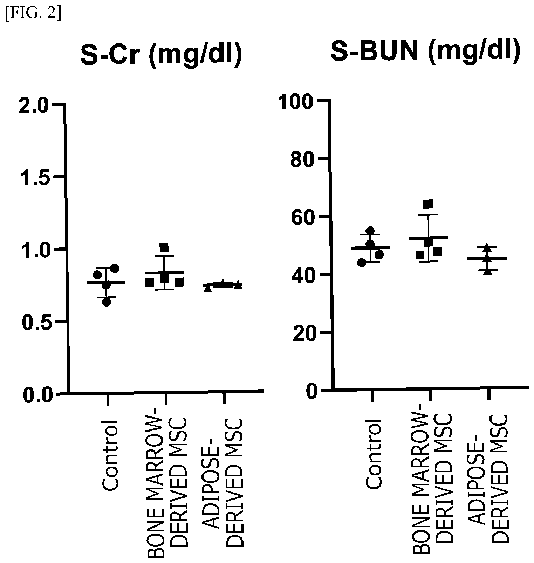

Anti-GBM nephritis was induced in rats (WKY/NCrj, female) in the same way as in Example 1. Then, 2 × 10⁶ bone marrow-derived mesenchymal stem cells (bone marrow-derived MSC group) or 2 × 10⁶ adipose-derived mesenchymal stem cells (adipose-derived MSCs) were administered immediately after the anti-GBM mouse IgG administration, on the second day and the fourth day. The cells were suspended in 2 mL of a starting solution (produced by Otsuka Pharmaceutical Factory, Inc.) including 5% (v/v) cryopreservation solution (STEM-CELLBANKER (R), GMP grade, containing 10% DMSO) and used for administration. The starting solution including 5% (v/v) cryopreservation solution was administered to a control group (control group). The serum creatinine concentration (S-Cr) and the blood urea nitrogen (S- BUN) were measured on the seventh day. Creatinine concentration increase, blood urea nitrogen increase, and crescent score increase due to anti-GBM nephritis were not suppressed by administering bone marrow-derived or adipose-derived MSCs in 5% (v/v) cryopreservation solution (Fig. 2).

### EXAMPLE 3

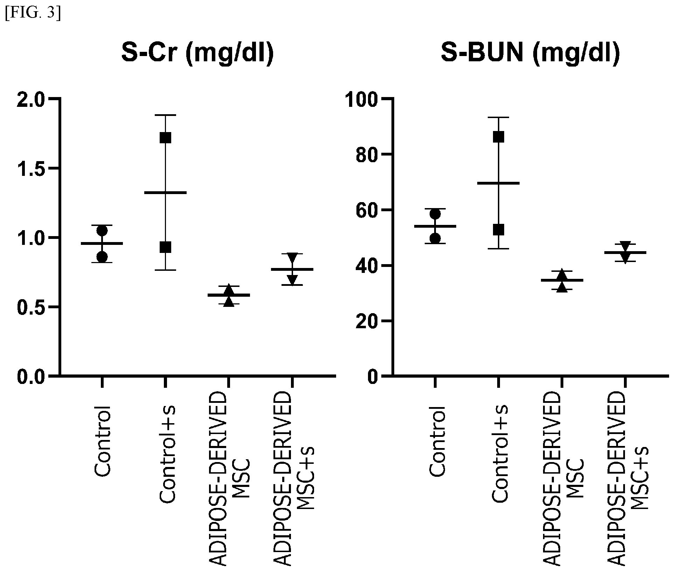

Anti--GBM nephritis was induced in rats (WKY/NCrj, female) in the same way as in Example 1. Then, 2 × 10⁶ adipose-derived mesenchymal stem cells (adipose-derived MSC group); 2 × 10⁶ adipose-derived mesenchymal stem cells with 5% (v/v) cryopreservation solution (STEM-CELLBANKER (R) GMP grade, containing 10% DMSO, adipose-derived MSC + S group); or 5% (v/v) cryopreservation solution (control + S group) was administered immediately after the anti-GBM mouse IgG administration, on the second day and the fourth day. The cells and the cryopreservation solution were suspended in 2 mL of a starting solution (produced by Otsuka Pharmaceutical Factory, Inc.) and used for administration, and only the starting solution was administered to a control group (control group). The serum creatinine concentration (S-Cr) and the blood urea nitrogen (S- BUN) were measured on the seventh day. Creatinine concentration increase, blood urea nitrogen increase, and crescent score increase due to anti-GBM nephritis were suppressed by administering the adipose-derived MSCs but slightly suppressed by administering the adipose-derived MSCs with 5% (v/v) cryopreservation solution (Fig. 3).

### EXAMPLE 4

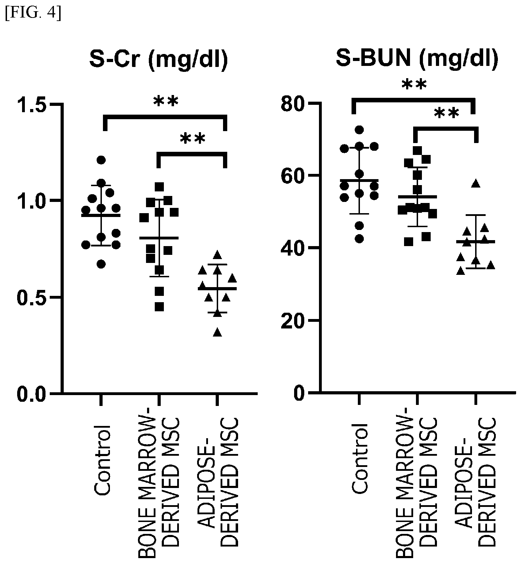

Anti-GBM nephritis was induced in rats (WKY/NCrj, female) in the same way as in Example 1. Then, 2 × 10⁶ bone marrow-derived mesenchymal stem cells (bone marrow-derived MSC group) or 2 × 10⁶ adipose-derived mesenchymal stem cells (adipose-derived MSCs) were administered immediately after the anti-GBM mouse IgG administration, on the second day and the fourth day. The cells were suspended in 2 mL of a starting solution (produced by Otsuka Pharmaceutical Factory, Inc.) including 1.65% (v/v) cryopreservation solution (STEM-CELLBANKER (R) GMP grade, containing 10% DMSO) and used for administration, and the starting solution including 1.65% (v/v) cryopreservation solution was administered to a control group (control group). The serum creatinine concentration (S-Cr) and the blood urea nitrogen (S- BUN) were measured on the seventh day. Creatinine concentration increase, blood urea nitrogen increase, and crescent score increase due to anti-GBM nephritis were suppressed by administering the adipose-derived MSCs in the 1.65% (v/v) cryopreservation solution (Fig. 4).

### EXAMPLE 5

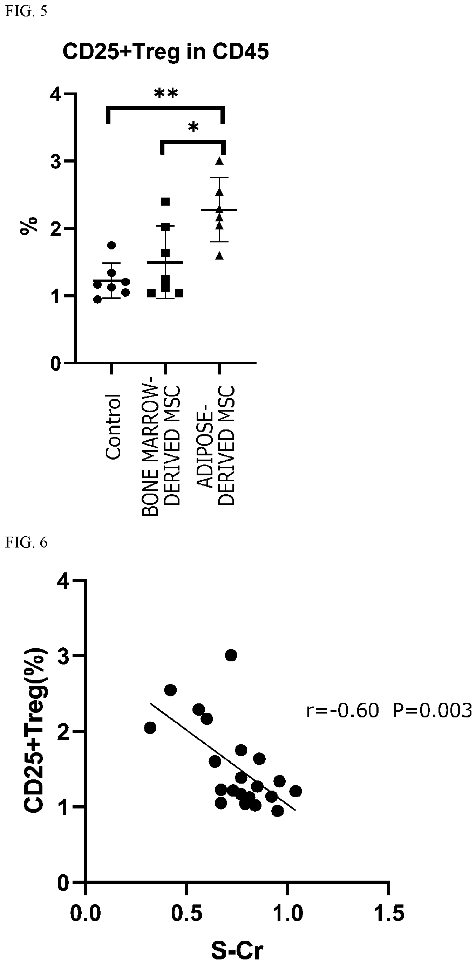

Anti-GBM nephritis was induced in rats (WKY/NCrj, female) in the same way as in Example 1. Then, 2 × 10⁶ bone marrow-derived mesenchymal stem cells (bone marrow-derived MSC group) or 2 × 10⁶ adipose-derived mesenchymal stem cells (adipose-derived MSCs) were administered immediately after the anti-GBM mouse IgG administration, on the second day and the fourth day. The cells were suspended in 2 mL of a starting solution (produced by Otsuka Pharmaceutical Factory, Inc.) including 1.65% (v/v) cryopreservation solution (STEM-CELLBANKER (R) GMP grade, containing 10% DMSO) and used for administration. And only the starting solution was administered to a control group (control group). The rate of CD25-positive Treg cells among CD45-positive cells in kidneys was measured, and the rate of CD25-positive Treg cells significantly increased in the adipose-derived MSC group (Fig. 5). The rate of CD25-positive Treg cells among CD45-positive cells in kidneys in the control group and the adipose-derived MSC group were measured, the serum creatinine concentration was compared and the negative correlation was confirmed. These results confirm that increasing the number of CD25-positive Treg cells in the kidney can reduce serum creatinine concentrations (Fig. 6).

### INDUSTRIAL APPLICABILITY

Since CD25-positive regulatory T cells in kidneys can be increased by an agent for increasing regulatory T cells of the present invention, the function, structure, and the like of disease sites of the kidney or the like can be improved remarkably. An excellent therapeutic effect is expected to be produced on renal diseases including glomerulonephritis such as IgA nephropathy thereby. Since the mesenchymal stem cells that the agent for increasing regulatory T cells of the present invention contains are hardly rejected by an allogeneic subject, cells obtained by subjecting donor cells prepared in advance to expansion culture and cryopreservation can be used. Therefore, it is also advantageous that the mesenchymal stem cells of the present invention are also easily commercialized compared to when autologous mesenchymal stem cells are prepared and used, and a certain stable effect can be easily obtained.

## Claims

1. An agent for increasing CD25-positive regulatory T cells in kidneys, comprising mesenchymal stem cells.

2. The agent for increasing CD25-positive regulatory T cells in kidneys according to claim 1, further comprising a cryopreservation solution at a concentration of less than 5% (v/v).

3. The agent for increasing CD25-positive regulatory T cells in kidneys according to claim 2, wherein the cryopreservation solution comprises DMSO (dimethyl sulfoxide).

4. The agent for increasing CD25-positive regulatory T cells in kidneys according to any one of claims 1 to 3, wherein the agent is for treating a renal disease.

5. The agent for increasing CD25-positive regulatory T cells in kidneys according to claim 4, wherein the renal disease is glomerulonephritis.
